# EUROPEAN PATENT APPLICATION

(11) **EP 3 025 678 A1**
(43) Date of publication of application: **01.06.2016**
(21) Application number: 14830326.6
(22) Date of filing: 23.07.2014
(51) Int. Cl.: A61F 2/16

(54) **INTRAOCULAR LENS-INSERTING INSTRUMENT**

(30) Priority: 24.07.2013 JP 2013153751
(71) Applicant: Kowa Company, Ltd., Nagoya-shi, Aichi-ken 460-8625 (JP)
(72) Inventor: SATO, Takashi, Nagoya-shi Aichi 457-0841 (JP)
(74) Representative: Dempster, Benjamin John Naftel
(86) International application number: PCT/JP2014/069457
(87) International publication number: WO 2015/012312

(57) **Abstract**

The present invention provides a technique that makes it possible, when inserting an intraocular lens into an eye by pushing a plunger, to insert a support positioned on the rear side of the pushing direction of the lens body of the intraocular lens integrally with the lens body and to stabilize the shape of the support after insertion. A slanted part (61f) slanted so as to be higher from the front side to the rear side of a plunger (60) or from the left side to the right side of a plunger (60) is provided at the tip (61d) of a plunger (60). A wall part (61g) formed in a plane perpendicular to the pushing direction of the plunger (60) is provided on the rear side of the slantedpart (61f). Aplate-shaped protrusion (61e) that protrudes forward from the slanted part (61f) so that part of the lens body can be mounted is provided on the lower side of the slanted part (61f).

## Description

### Technical Field

The present invention relates to an intraocular lens-inserting instrument to insert an intraocular lens into a patient's eyeball.

### Background Art

In a conventional operation of cataract or the like, treatment of making an incision in an eye tissue such as the cornea or anterior capsule of crystalline lens in the eyeball, extracting and removing the crystalline lens in the capsule through the incision, and then inserting an intraocular lens replacing the crystalline lens into the eye through the incision and arranging the intraocular lens inside the capsule is provided.

Particularly in recent years, an inserting instrument as shown below is frequently used when an intraocular lens is inserted into an eyeball through an incision. That is, a tip opening of an insertion cylindrical portion provided in a tip portion of an instrument main body is inserted into the eyeball through the incision and also an intraocular lens deformed so as to be smaller inside the instrument main body is pushed out from the tip opening of the insertion cylindrical portion by a rod-like plunger to discharge and insert the intraocular lens into the eyeball. By using such the inserting instrument, the size of an incision can be made smaller and also the intraocular lens can be easily inserted into the eyeball therefore, burdens in operation can be reduced. In addition, the intraocular lens can easily be inserted into the eyeball using the incision formed to extract and remove the crystalline lens and therefore, the operation can be made simpler and astigmatism and infectious diseases after the operation can be inhibited from developing.

The aforementioned intraocular lens is frequently composed of a lens body having predetermined optical performance and two whisker-like supports to hold the lens body inside the eyeball and when the intraocular lens is inserted into the eyeball from the inserting instrument, the two supports are frequently arranged backward and forward the lens body during insertion. Then, it is sometimes difficult to integrally handle particularly the support arranged backward with the lens body due to unexpected deformation in insertion work of the intraocular lens. As a result, it is difficult to control the shape of the support during insertion of the intraocular lens into the eyeball and after the intraocular lens is released into the eye and it takes time to adjust the support shape to enable the support to support the lens body inside the eyeball in a stable manner, which sometimes decreases the efficiency of insertion work of the intraocular lens into the eyeball.

### Citation List

### Patent Literature

PTL 1: JP 08-024282 A
PTL 2: JP 2009-028223 A

### Summary of Invention

### Technical Problem

The present invention is devised in view of the above problem of the conventional technology and an object thereof is to provide a technology capable of inserting, when an intraocular lens is inserted into an eyeball by pressing the lens by a plunger, a support arranged on the backward side in a pressing direction of a lens body of the intraocular lens integrally with the lens body and stabilizing the shape of the support after the insertion.

In order to solve the above issues, the greatest characteristic of the present invention is that an inclined plane inclined toward an optical axis front side of the lens body from a front side to the back side in the pressing direction of a plunger is provided at the tip of the plunger, and when the intraocular lens is pressed by the tip of the plunger, the support arranged on the back side in the pressing direction of the lens body is caused to be deformed to the optical axis front side of the lens body by the inclined plane and also the support is deformed by the inclined plane to the front side in the pressing direction to be able to be folded up such that the support overlaps with the surface on the optical axis front side of the lens body when viewed from an optical axis side.

More specifically, an intraocular lens-inserting instrument that inserts an intraocular lens into an eyeball by releasing the intraocular lens into the eyeball from a tip of an insertion cylindrical portion inserted into the eyeball through an incision, includes: an instrument main body having the insertion cylindrical portion inserted through the incision formed in an eyeball tissue and formed into a substantially tubular shape; an housing portion provided integrally with or separately from the instrument main body and capable of arranging the intraocular lens in the instrument main body by accommodating the intraocular lens including a lens body and a plurality of supports that support the lens body in the eyeball; and a plunger that releases the intraocular lens from a tip of the insertion cylindrical portion into the eyeball by pressing the intraocular lens accommodated in the housing portion by the tip by being pushed into the instrument main body, wherein the intraocular lens is arranged in the instrument main body such that one support is arranged on a back side in a pressing direction of the lens body by the plunger, an inclined plane inclined toward an optical axis front side of the lens body from a front side to the back side in the pressing direction is provided at the tip of the plunger, and when the intraocular lens is pressed by the tip of the plunger, the tip of the support arranged on the back side in the pressing direction of the lens body is caused to be arranged on the optical axis front side of a surface on the optical axis front side of the lens body by deforming the support to the optical axis front side of the lens body by the inclined plane and also the support is deformed by the inclined plane to the front side in the pressing direction to be able to be folded up such that the support overlaps with the surface on the optical axis front side of the lens body when viewed from an optical axis side.

### Solution to problem

1 Accordingly, when the intraocular lens is released into the eyeball by pressing the intraocular lens by the tip of the plunger, the support arranged on the back side in the pressing direction of the lens body is first deformed to the lens optical axis front side and also deformed to the front side in the pressing direction, by bringing the support into contact with the inclined plane at the tip of the plunger, to be able to overlap with the surface on the optical axis front side of the lens body of the intraocular lens when viewed from the optical axis direction. When the intraocular lens is moved to the front side of the instrument main body by pressing the lens body of the intraocular lens by the tip of the plunger, the lens body is deformed substantially cylindrically around the traveling direction in accordance with changes of the cross section area of the instrument main body. In such a situation, when the intraocular lens is subsequently released into the eyeball by pressing the lens body of the intraocular lens by the tip of the plunger, according to the present invention, the lens body is further deformed while the support arranged on the back side in the pressing direction of the lens body is made to overlap with the lens body when viewed from the optical axis direction of the lens body so that the intraocular lens can be released into the eyeball in a state in which the support is enveloped in the lens body.

Then, when the intraocular lens is released into the eyeball, the shape of the support on the back side in the pressing direction of the lens body can be stabilized so that the shape of the support can be inhibited from varying inside the eyeball. As a result, after the intraocular lens is released into the eyeball, postures of the lens body and the support inside the eyeball can be adjusted more efficiently.

In addition, in the present invention, the inclined plane may be inclined toward the optical axis front side of the lens body from a root side to a tip side of the support arranged on the back side in the direction perpendicular to the pressing direction and the optical axis of the lens body. Then, the support arranged on the back side in the pressing direction of the lens body can be deformed more reliably by the tip of the plunger so as to be inclined toward the optical axis front side of the lens body from the root to the tip side thereof. Then, the tip of the support can be arranged on the optical axis front side of the lens body more reliably and subsequently, simply by deforming the support to the front side in the pressing direction, the support can be folded up on the surface on the front side in the optical axis direction of the lens body such that the support overlaps with the surface on the optical axis front side of the lens body when viewed from the optical axis side more reliably.

In addition, in the present invention, a wall portion formed perpendicularly with respect to the pressing direction to move the lens body to the front side in the pressing direction and also to deform the support arranged on the back side in the pressing direction of the lens body to the front side in the pressing direction may be provided on the back side in the pressing direction of the inclined plane at the tip of the plunger.

Then, when the intraocular lens is inserted, the support arranged on the back side in the pressing direction of the lens body can be deformed to the front side in the pressing direction more reliably and also the intraocular lens can be released into the eyeball from the instrument main body more reliably while the support is folded up on the surface on the front side in the optical axis direction of the lens body such that the support overlaps with the surface when viewed from the optical axis side. "Formed perpendicularly with respect to the pressing direction" above does not mean complete perpendicularity and means an angle allowing the lens body to move to the front side in the pressing direction in a stable manner and also allowing the support arranged on the back side in the pressing direction of the lens body to be deformed to the front side in the pressing direction in a stable manner. The expression is intended to include an angle in the range of about ±20° with respect to the complete perpendicularity.

In addition, in the present invention, a recess that accommodates a linking portion of the support that is folded up and the lens body in a state in which the support arranged on the back side in the pressing direction of the lens body is folded up on the surface on the optical axis front side of the lens body so as to overlap therewith when viewed from the optical axis side may be provided at the tip of the plunger.

That is, while the support arranged on the back side in the pressing direction of the lens body is folded up on the surface on the optical axis front side of the lens body such that the support overlaps with the surface when viewed from the optical axis side, if the intraocular lens is viewed from the optical axis front side, a portion around the linking portion of the folded support and the lens body projects from an outer shape of the lens body. Then, when the lens body is pressed by the tip of the plunger, there is the possibility that the portion around the linking portion of the support and the lens body is caught between the plunger tip and the inner wall of the insertion cylindrical portion or an insertion resistance increases after the linking portion is brought into contact with the inner wall of the insertion cylindrical portion strongly.

In the present invention, by contrast, a recess accommodating the linking portion of the support and the lens body is provided at the tip of the plunger and then, when the lens body is pressed by the tip of the plunger, the portion around the linking portion of the support and the lens body can be inhibited from being caught in a gap between the plunger tip and the inner wall of the insertion cylindrical portion and also the insertion resistance can be inhibited from increasing after the linking portion is brought into contact with the inner wall of the insertion cylindrical portion strongly so that the intraocular lens can be released into the eyeball more smoothly.

Inaddition, in the present invention, a projecting portion projecting to the front side in the pressing direction from the inclined plane to make a portion of the lens body mountable thereon may be provided on an optical axis back side of the lens body of the inclined plane at the tip of the plunger.

Accordingly, when the intraocular lens is pressed by the plunger, the lens body can be brought into contact with the tip of the plunger in a stable manner. Therefore, when the intraocular lens is released from the insertion cylindrical portion into the eyeball, inconveniences such as the lens body being caught between the instrument main body and the plunger after the lens body comes off the tip of the plunger can be inhibited more reliably. As a result, when the intraocular lens is released from the tip of the insertion cylindrical portion into the eyeball, the posture of the intraocular lens can be stabilized so that the intraocular lens can be released into the eyeball more smoothly.

If the tip of the insertion cylindrical portion of the instrument main body is obliquely cut such that a region on the optical axis front side of the lens body is more forward in the pressing direction of the intraocular lens, when the intraocular lens is released from the tip of the insertion cylindrical portion by being pressed by the tip of the plunger, the intraocular lens can be released in a state held by a region on the front side in the pressing direction at the tip of the insertion cylindrical portion and the projecting portion of the plunger. Then, the posture of the lens body can be stabilized still more when the intraocular lens is released. As a result, the intraocular lens can be released into the eyeball still more smoothly.

In addition, in the present invention, the inclined plane may be provided such that an end on the optical axis front side of the inclined plane is arranged on the optical axis front side from an end face of the surface on the optical axis front side of the lens body. Then, the tip of the support arranged on the back side in the pressing direction of the lens body can be moved from the end face of the surface on the optical axis front side of the lens body to the optical axis front side more reliably and so that the support can more reliably be folded up on the surface on the optical axis front side of the lens body such that the support overlaps with the surface when viewed from the optical axis side.

Solutions to the problem of the present invention described above can be used in combination as long as possible.

### Advantageous Effects of Invention

According to the present invention, when an intraocular lens is inserted into an eyeball by pressing the lens by a plunger, a support arranged on the backward side in a pressing direction of a lens body of the intraocular lens can be inserted integrally with the lens body and the shape of the support after the insertion can be stabilized.

### Brief Description of Drawings

FIG. 1 is a diagram showing an outline configuration of a conventional intraocular lens-inserting instrument.
FIG. 2 is a diagram showing an outline configuration of an intraocular lens.
FIG. 3 is a diagram showing an outline configuration of a conventional nozzle body.
FIG. 4 is a diagram showing an outline configuration of a conventional positioning member.
FIG. 5 is a diagram showing an outline configuration of a conventional plunger.
FIG. 6 is a diagram showing around the tip of a plunger according to a first embodiment of the present invention.
FIG. 7 is a diagram illustrating the action of the plunger according to the first embodiment of the present invention.
FIG. 8 is a diagram illustrating a height relationship of the tip of the plunger and the intraocular lens according to the first embodiment of the present invention.
FIG. 9 is a diagram showing around the tip of a plunger according to a second embodiment of the present invention.
FIG. 10 is a diagram showing around the tip of a plunger according to a third embodiment of the present invention.

### Description of Embodiments

Hereinafter, the embodiments of the present invention will be described with reference to the drawings.

### <First embodiment>

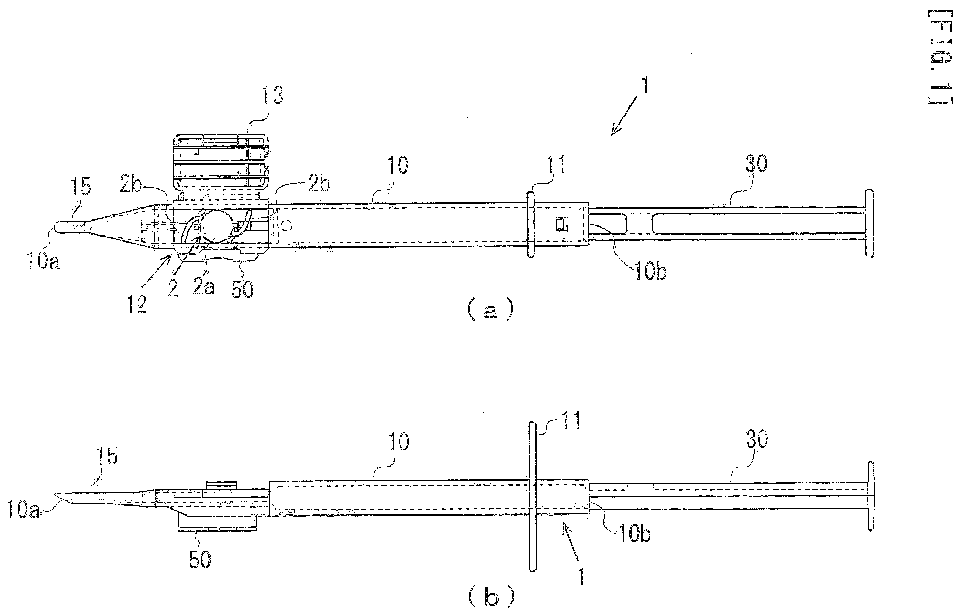

FIG. 1 shows an outline configuration of a conventional intraocular lens-inserting instrument 1 (hereinafter, may simply called the inserting instrument 1). FIG. 1(a) shows a plan view and FIG. 1 (b) shows a side view. The inserting instrument 1 is formed in a tubular shape with a substantially rectangular cross section and a wide opening on one side (hereinafter, the side with a wide opening will be called a back end portion 10b) and includes a nozzle body 10 as an instrument main body including a nozzle portion 15 as an insertion cylindrical portion thinly narrowed down and a tip portion 10a open obliquely on the other end and a plunger 30 that can reciprocate after being inserted into the nozzle body 10. Hereinafter, the direction from the back end portion 10b to the tipportion 10a of the nozzle body 10 will be called a forward direction, the opposite direction thereof will be called a backward direction, the frontward side of FIG. 1 (a) will be called an upward direction, the opposite direction thereof will be called a downward direction, the frontward side of FIG. 1 (b) will be called a left direction, and the opposite direction thereof will be called a right direction. In this case, the upper side corresponds to an optical axis front side of a lens body 2a described below, the downward side corresponds to an optical axis back side of the lens body 2a, the front side corresponds to the front side in the pressing direction by the plunger 30, and the back side corresponds to the back side in the pressing direction by the plunger 30.

A hold portion 11 that tabularly rises and on which the user puts his (her) finger when pushing the plunger 30 to the tip side of the nozzle body 10 is integrally provided near the back end portion 10b of the nozzle body 10. Also, a stage portion 12 as an housing portion that sets an intraocular lens 2 is provided on the back side of the nozzle portion 15 in the nozzle body 10. The stage portion 12 is set such that the upper side of the nozzle body 10 is opened by opening a stage cover portion 13. In addition, a positioning member 50 is mounted on the stage portion 12 from below the nozzle body 10. Thanks to the positioning member 50, the intraocular lens 2 is held in a stable manner inside the stage portion 12 before use (during transportation).

That is, in the inserting instrument 1, the intraocular lens 2 is set to the stage portion 12 with the optical axis front side directed upward while the stage cover portion 13 is open and the positioning member 50 is mounted on the stage portion 12 during production. Then, after the stage cover portion 13 is closed, the inserting instrument 1 is shipped and sold. Further, the user removes the positioning member 50 while the stage cover portion 13 is closed for use and then pushes the plunger 30 to the tip side of the nozzle body 10. This presses and moves the intraocular lens 2 up to the nozzle portion 15 through the plunger 30 and then, the intraocular lens 2 is released into the eyeball from the tip portion 10a. The nozzle body 10, the plunger 30, and the positioning member 50 in the inserting instrument 1 are formed from a resin material such as polypropylene. Polypropylene is a material that has a track record in medical equipment and high reliability of chemical resistance.

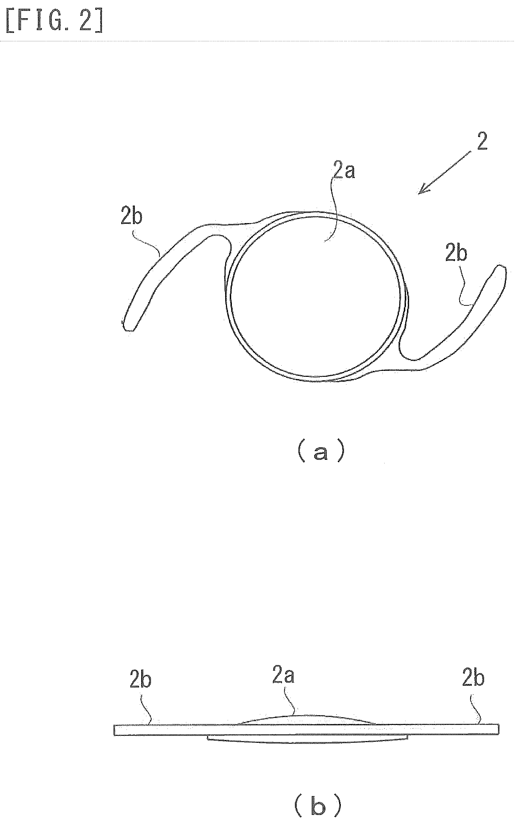

FIG. 2 is a diagram showing an outline configuration of the intraocular lens 2. FIG. 2(a) shows a plan view and FIG. 2 (b) shows a side view. The intraocular lens 2 is composed of a lens body 2a having predetermined refracting power and two whisker-like supports 2b, 2b to hold the lens body 2a inside the eyeball. The lens body 2a and the support 2b are formed from flexible resin material. In the inserting instrument 1 according to the present embodiment, the intraocular lens 2 is set such that one of the two supports 2b is arranged on the back side of the lens body 2a and the other is arranged on the front side of the lens body 2a.

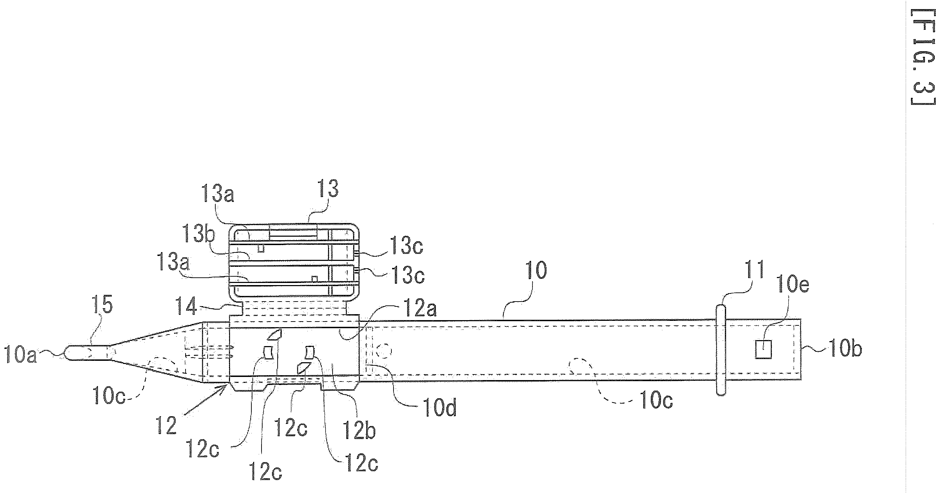

FIG. 3 shows a plan view of the nozzle body 10. In the nozzle body 10, as described above, the intraocular lens 2 is set to the stage portion 12. Then, in this setting, the intraocular lens 2 is pressed by the plunger 30 and released from the tip portion 10a. A through hole 10c whose sectional shape changes in accordance with a change of an outside shape of the nozzle body 10 is provided inside the nozzle body 10. Then, when the intraocular lens 2 is released, the intraocular lens 2 is deformed in accordance with a change of the sectional shape of the through hole 10c inside the nozzle body 10 so that the intraocular lens 2 is released after being deformed into a shape that can enter an incision formed on the patient's eyeball more easily.

The tip portion 10a has a shape obtained by oblique cutting such that an upper region of the nozzle portion 15 is on the front side of a lower region thereof. The obliquely cut shape of the tip portion 10a may be linear oblique cutting when viewed from the left and right direction or oblique cutting with swelling outward, that is, in a curved surface shape.

A stage groove 12a having a width slightly larger than a diameter of the lens body 2a of the intraocular lens 2 is formed in the stage portion 12. The dimension of the stage groove 12a in a forward and backward direction is set larger than the dimension of the maximum width including the supports 2b, 2b extending to both sides of the intraocular lens 2. Also, a set surface 12b is formed by the bottom of the stage groove 12a. The position of the set surface 12b in the upward and downward direction is set above the height position of the bottom of the through hole 10c of the nozzle body 10 and the bottoms of the set surface 12b and the through hole 10c are linked by a bottom slanted surface 10d.

The stage portion 12 and the stage cover portion 13 are formed integrally. The stage cover portion 13 has a dimension in the forward and backward direction similar to that of the stage portion 12. The stage cover portion 13 is linked by a coupling portion 14 in a thin plate shape formed by a side face of the stage portion 12 being extended to the side of the stage cover portion 13. The coupling portion 14 is formed bendably in a center portion and the stage cover portion 13 can overlap with the stage portion 12 from above by bending the coupling portion 14 to close the cover.

Ribs 13a, 13b are provided on a surface opposite to the set surface 12b when the cover is closed in the stage cover portion 13 to reinforce the stage cover portion 13 and to stabilize the position of the intraocular lens 2. Also, a guide protrusion 13c is provided as an upper-side guide of the plunger 30.

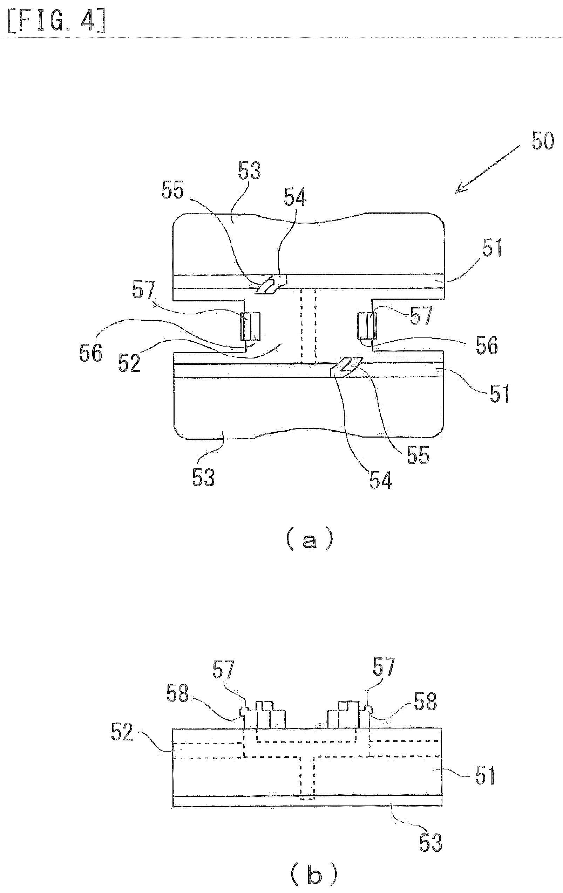

The positioning member 50 is removably mounted on the downward side of the set surface 12b of the stage portion 12. FIG. 4 shows an outline configuration of the positioning member 50. FIG. 4 (a) shows a plan view and FIG. 4 (b) shows the left side view. The positioning member 50 is configured separately from the nozzle body 10 and structured to have a pair of sidewall portions 51, 51 linked by a coupling portion 52. Holdingportions 53, 53 extending and stretching outward are formed on the downward end of the sidewall portions 51.

Then, a pair of first mounting portions 54, 54 whose shape when viewed from above is circular and projecting upward is formed in an upper end portion of the respective sidewall portions 51, 51. Further, first positioning portions 55, 55 are formed on an outer circumferential side on an upper end surface of the first mounting portions 54 by projecting. The distance between internal circles of the first positioning portions 55, 55 is set slightly larger than the diameter dimension of the lens body 2a of the intraocular lens 2.

A pair of second mounting portions 56, 56 whose shape when viewed from above is rectangular and projecting upward is formed on both sides in the forward and backward direction of the coupling portion 52. The height of the top surface of the second mounting portions 56, 56 is equal to the height of the top surface of the first mounting portions 54, 54. Further, second positioning portions 57, 57 further projecting upward wholly in the left and right direction of the second mounting portions 56, 56 are formed in an outer portion of the top surface of the second mounting portions 56, 56. The distance between the inner sides of the second positioning portions 57, 57 is set slightly larger than the diameter dimension of the lens body 2a of the intraocular lens 2. In addition, locking claws 58, 58 projecting slightly in the forward and backward direction are formed wholly in the left and right direction in an upper end portion of the second mounting portions 56, 56.

The above positioning member 50 is mounted from below the set surface 12b of the nozzle body 10. A set surface through hole 12c passing through the set surface 12b in a thickness direction is formed in the set surface 12b of the nozzle body 10. The outer shape of the set surface through hole 12c is a substantially similar shape slightly larger than a shape when the first mounting portions 54, 54 and the second mounting portions 56, 56 of the positioning member 50 are viewed from above. Then, when the positioning member 50 is mounted on the nozzle body 10, the first mounting portions 54, 54 and the second mounting portions 56, 56 are inserted through the set surface through hole 12c from below the set surface 12b to project above the set surface 12b.

At this point, the locking claws 58, 58 provided in the second positioning portions 57, 57 project to the set surface 12b via the set surface through hole 12c to be locked onto the top surface of the set surface 12b. In this manner, the positioning member 50 is mounted from below the nozzle body 10 and the first mounting portions 54, 54 and the second mounting portions 56, 56 are fixed while projecting from the set surface 12b. Then, when the intraocular lens 2 is set to the set surface 12b, the bottom of the outer circumferential portion of the lens body 2a is placed on the top surface of the first mounting portions 54, 54 and the second mounting portions 56, 56. Theposition of the lens body 2a is regulated in a horizontal direction by the first positioning portions 55, 55 and the second positioning portions 57, 57.

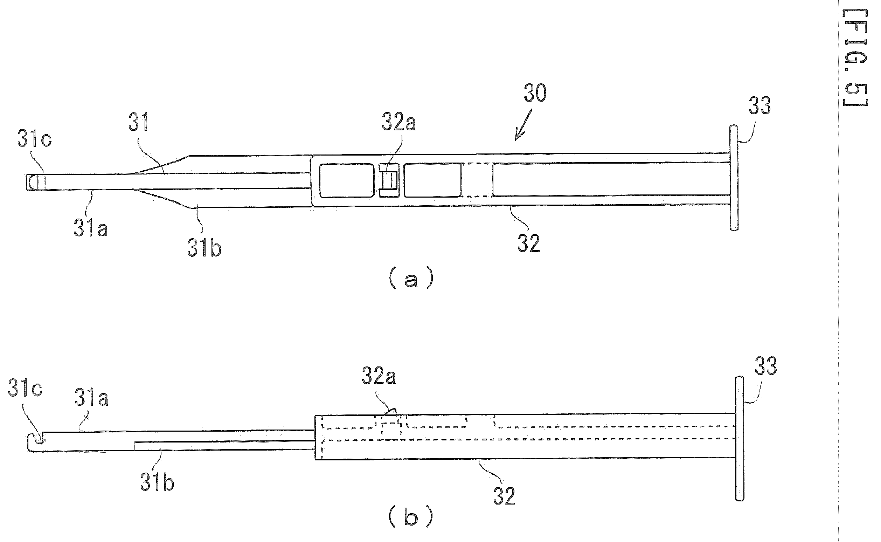

FIG. 5 shows an outline configuration of the conventional plunger 30. The plunger 30 has a length in the forward and backward direction slightly larger than that of the nozzle body 10. The plunger 30 is formed of an action portion 31 on the tip side whose shape is basically cylindrical and an insertion portion 32 on the back end side in a basically rectangular rod shape. The action portion 31 includes a cylindrical portion 31a in a cylindrical shape and a flat portion 31b in a thin plate shape stretching in the left and right direction of the cylindrical portion 31a.

A notch portion 31c is formed in a tip portion of the action portion 31. As is evident from FIG. 5, the notch portion 31c is formed like a groove open in the upward direction of the action portion 31 and passing through in the left and right direction. Also, as is evident from FIG. 5 (b), the groove wall on the tip side of the notch portion 31c is formed of an inclined plane directed upward as the tip of the action portion 31 becomes closer.

On the other hand, the insertion portion 32 has a substantially H-shaped cross section as a whole and dimensions in the left and right direction and the upward and downward direction thereof are set slightly smaller than those of the through hole 10c of the nozzle body 10. Also, a disc-like pressing plate portion 33 stretching in the upward and downward direction and the left and right direction is formed at the back end of the insertion portion 32

A claw portion 32a projecting upward from the insertion portion 32 and movable upward and downward due to elasticity of the material of the plunger 30 is formed in a forward portion from the center in the forward and backward direction of the insertion portion 32. When the plunger 30 is inserted into the nozzle body 10, a locking hole 10e provided in a thickness direction as shown in FIG. 3 and the claw portion 32a are engaged on the top surface of the nozzle body 10, thereby determining the relative position of the nozzle body 10 and the plunger 30 in the initial state. The formation positions of the claw portion 32a and the locking hole 10e are set in an engaged state such that the tip of the action portion 31 is positioned in a rear direction of the lens body 2a of the intraocular lens 2 set to the stage portion 12 and the notch portion 31c is located in a position where the support 2b on the back side of the lens body 2a can be supported frombelow.

Before the intraocular lens 2 is accommodated in the inserting instrument 1 configured as described above, the plunger 30 is inserted into the nozzle body 10 and arranged in an initial position. Also, the positioning member 50 is mounted, as described above, on the nozzle body 10 from below the set surface 12b. Accordingly, the first mounting portions 54, 54 and the second mounting portions 56, 56 of the positioning member 50 are held in a projecting state from the set surface 12b.

Next, the lens body 2a of the intraocular lens 2 in a state in which the supports 2b, 2b are directed in the forward and backward direction of the nozzle body 10 is mounted on the top surface of the first mounting portions 54, 54 and the second mounting portions 56, 56 for positioning. In this state, the outer circumferential portion of the lens body 2a is in contact with the first mounting portions 54, 54 and the second mounting portions 56, 56 and thus, the center portion of the intraocular lens 2 is supported in a no load state. Also in this state, the support 2b on the back side of the intraocular lens 2 is supported by the bottom of the notch portion 31c of the plunger 30.

When the intraocular lens 2 is inserted into an eyeball using the inserting instrument 1, the positioning member 50 is first removed from the nozzle body 10. Accordingly, the first mounting portions 54, 54 and the second mounting portions 56, 56 supporting the lens body 2a of the intraocular lens 2 recede from the set surface 12b and the intraocular lens 2 is movably mounted on the set surface 12b. Then, the intraocular lens 2 is pushed up to a predetermined position by the plunger 30.

Subsequently, the tip portion 10a in the nozzle portion 15 of the nozzle body 10 is inserted through an incision provided in the eye tissue. Here, the tip portion 10a has an oblique opening shape and can easily be inserted through the incision. Then, after the nozzle portion 15 is inserted through the incision, the pressing plate portion 33 of the plunger 30 is pushed to the tip side of the nozzle body 10 again in this state. Accordingly, the tip of the action portion 31 of the plunger 30 comes into contact with the outer circumference of the lens body 2a of the intraocular lens 2 set to the set surface 12a so that the intraocular lens 2 is guided toward the tip portion 10a by the plunger 30.

In operations in which the intraocular lens 2 is inserted into the patient's eyeball using the conventional intraocular lens-inserting instrument 1 as described above, the support 2b on the back side of the lens body 2a may unexpectedly be deformed so that a stable release of the intraocular lens 2 into the eyeball is prevented by being caught between the action portion 31 of the plunger 30 and the through hole 10c or the like. Also, when the intraocular lens 2 is released into the eyeball, it may become difficult to normally support the lens body 2a due to deformation of the support 2b on the back side. In such cases, it is necessary for an operator to make adjustments so that the support 2b is in a desirable posture after the intraocular lens 2 being released into the eyeball, which may decrease the efficiency of insertion operations of the intraocular lens 2.

As a countermeasure, inhibiting unexpected deformation of the support 2b by pressing both of the support 2b on the back side and the lens body 2a by the tip of the plunger 30 can be considered, but this alone could lead to inconveniences such as contact and adsorption of the support 2b on the back side of the lens body 2a by the outer circumferential portion of the lens body 2a or being slipped under the lens body 2a

Thus, in the present embodiment, the tip of the plunger is provided with an inclined portion and the support 2b on the back side of the lens body 2a is first pressed by the inclined portion to deform the support 2b upward and further, the support 2b is pressed forward to fold up the support 2b on the upper surface (front surface) of the lens body 2a. Then, the lens body 2a is pressed forward by the tip of the plunger to deform the lens body 2a so as to be convex downward in accordance with the sectional shape of the through hole 10c of the nozzle body 10 while the support 2b is folded up on the lens body 2a. In this way, the support 2b folded up on the lens body 2a is further enveloped and in this state, the intraocular lens 2 is released into the eyeball.

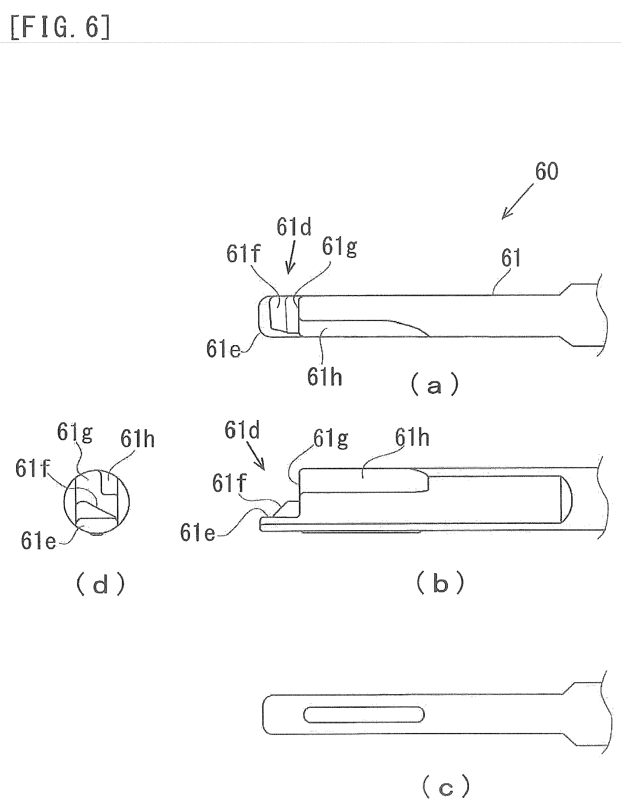

FIG. 6 shows a schematic view of a tip portion of a plunger 60 according to the present embodiment. FIG. 6(a) is a plan view of the tip portion of the plunger 60, FIG. 6(b) is the left side view, FIG. 6 (c) is a bottom view, and FIG. 6(d) is a front view when viewed from the front side.

In the present embodiment, as shown in FIG. 6, a protrusion 61e in a flat plate shape as a projecting portion where the lens body 2a is mounted is provided below a tip 61d of an action portion 61, that is, on the optical axis back side of the lens body 2a. Then, an inclined portion 61f as an inclined plane to deform the support 2b on the back side of the lens body 2a upward is provided on the protrusion 61e, that is, on the optical axis front side of the lens body 2a. Further, a wall portion 61g provided in a direction perpendicular to the pressing direction by the plunger 60 to press forward the support 2b deformed upward by the inclined portion 61f to deform the support 2b so as to overlap with an upper surface (front surface) of the lens body 2a when viewed from the upward and downward direction is provided on the back side of the protrusion 61e and the inclined portion 61f.

The inclined portion 61f in the present embodiment is formed such that the height thereof increases from the front side to the back side of the plunger 60 and also increases from the left side to the right side of the plunger 60. This corresponds to the fact that the inclined portion 61f is inclined toward the optical axis front side of the lens body from the front side to the back side in the pressing direction by the plunger 60 and also toward the optical axis front side of the lens body from the root to the tip side of the support arranged on the back side in the pressing direction of the lens body. This enables to deform the support 2b on the back side of the lens body 2a more easily such that the tip side is higher than the root. If the direction in which the support 2b on the back side of the lens body 2a extends is opposite to the direction shown in FIG. 2, that is, if the support 2b extends clockwise from the lens body 2a, the inclined portion 61f is formed such that the height thereof increases from the right side to the left side of the plunger 60.

A support relief groove 61h is provided at the tip 61d of the plunger 60 according to the present embodiment. When the support 2b on the back side of the lens body 2a is folded up on the upper surface of the lens body 2a, a portion near the linking portion of the support 2b and the lens body 2a projects from the outer circumference of the lens body 2a and the support relief groove 61h is a recess provided to inhibit the projecting portion from being caught between the plunger 60 and the through hole 10c.

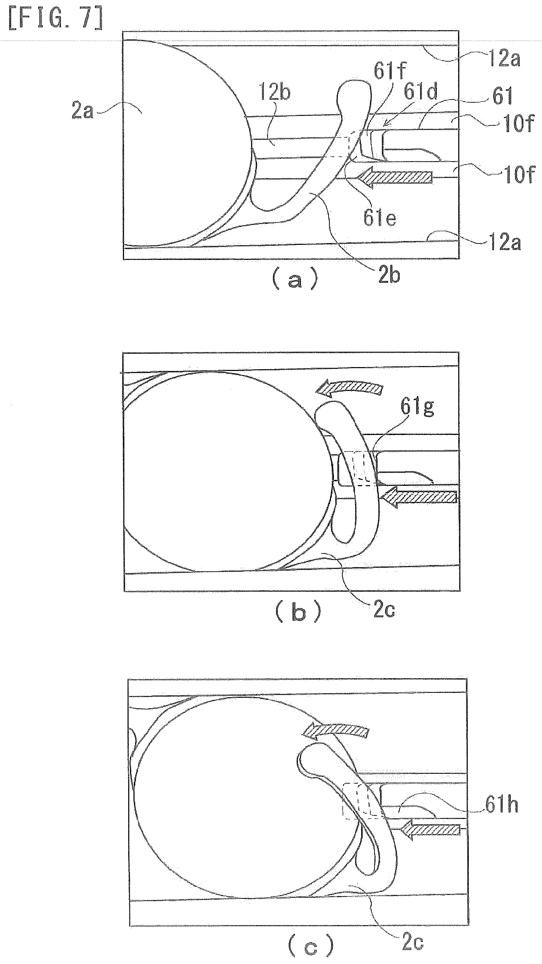

Next, the operation in the present embodiment will be described using FIG. 7. In FIG. 7, a case in which mound-like rails 10f, 10f to regulate the position in the left and right direction of the plunger 60 are provided on the set surface 12b as the bottom of the stage portion 12 of the nozzle body 10 will be described. FIG. 7 (a) shows a state when pushing of the plunger 60 is started from the initial state. In the present embodiment, the support 2b on the back side of the lens body 2a is mounted on the tip 61d of the plunger 60 and supported in the initial state of the inserting instrument 1.

Then, when the plunger 60 is started to move forward by the plunger 60 being pushed by the operator, the support 2b is brought into contact with the inclined portion 61f and further, the support 2b is pushed upward to start deformation by the inclined portion 61f being moved forward. The inclined portion 61f is formed so as to rise from the front side to the back side of the plunger 60 and also to rise from the left side to the right side of the plunger 60 and thus, the support 2b is more easily deformed to rise from the root to the tip side.

In FIG. 7(b), the support 2b is deformed upward by being brought into contact with the inclined portion 61f and the support 2b is also deformed forward by being pressed by the wall portion 61g and the tip thereof is close to the lens body 2a. In FIG. 7 (c), the support 2b is further deformed and the tip of the support 2b overlaps with the upper surface (front surface) of the lens body 2a. The lens body 2a is mounted on the rails 10f, 10f and thus, there is a gap between the lens body 2a and the set surface 12b. In this state, therefore, the protrusion 61e at the tip 61d of the plunger 60 get into the space between the back surface of the lens body 2a and the set surface 12b. If the pushing operation of the plunger 60 is further continued from this state, the intraocular lens 2 is moved forward inside the nozzle body 10 by the support 2b and the lens body 2a being pressed forward by the wall portion 61g and the inclined portion 61f. Then, when passing through the nozzle portion 15, the lens body 2a is deformed so as to be convex downward and released into the eyeball by enveloping the support 2b overlapping with the upper surface of the lens body 2a.

Then, the lens body 2a is restored to its initial shape by the elastic force thereof inside the eyeball and with such an operation, the support 2b enveloped in the lens body 2a is restored to its original posture as shown in FIG. 2. Thus, according to the present embodiment, by a simple operation of pushing the plunger 60, the support 2b on the back side of the lens body 2a is deformed on the upper side and the front side to be folded up so as to overlap with the upper surface of the lens body 2a and then, the lens body 2a is deformed so as to be convex downward to be able to integrally release the lens body 2a and the support 2b into the eyeball. Therefore, inconveniences such as wasting time to adjust the posture of the support after the insertion without being able to control the posture of particularly the support on the back side during insertion of the intraocular lens into an eyeball can be avoided.

When the intraocular lens 2 is released into the eyeball, a linking portion 2c of the deformed support 2b and the lens body 2a projects like a protrusion to the back side from the outer shape of the lens body 2a deformed so as to be convex downward and the linking portion 2c gets into the support relief groove 61h and is accommodated and thus, inconveniences such as the linking portion 2c being caught between the action portion 61 of the plunger 60 and the through hole 10c of the instrument main body 10 can be inhibited from occurring. Accordingly, the intraocular lens 2 can be released into the eyeball more smoothly.

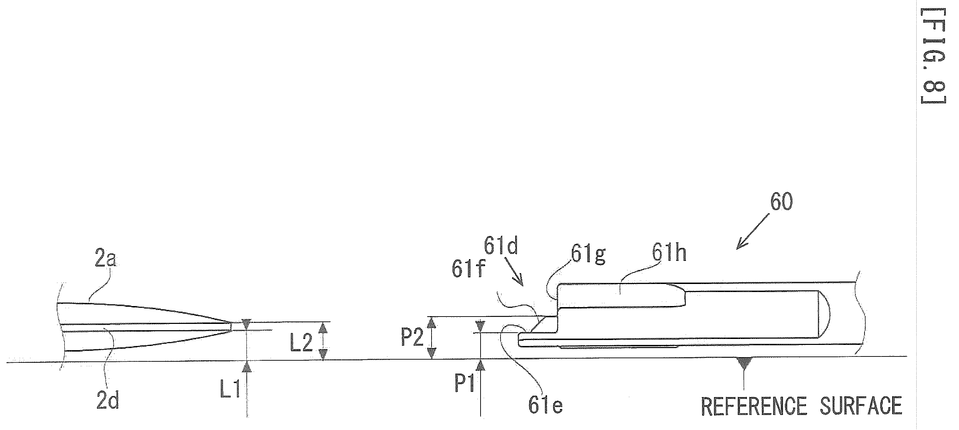

In the present embodiment, as shown in FIG. 8, the dimensional relationship is set such that a height P2 from a predetermined reference surface to the upper end of the inclined portion 61f is higher than a height L2 of the front surface end of the intraocular lens 2 in a state of being mounted on the stage portion 12 after the positioning member 50 being removed from the reference surface, that is, P2 > L2 applies. Then, when the support 2b on the back side of the lens body 2a is deformed upward by being brought into contact with the inclined portion 61f and further deformed forward by being brought into contact with wall portion 61g by pushing the plunger 60, without interfering with a lens end face 2d, the tip of the support 2b can be folded up so as to overlap with the upper surface of the lens body 2a more reliably.

The heights P2, L2 from the reference surface are heights from a predetermined position when the instrument main body 10 is mounted with the plunger 60 and the intraocular lens 2 and when, for example, the action portion 61 of the plunger 60 and the intraocular lens 2 are mounted on the same plane, the mounted plane may be used as the reference surface. In such a case, P2 is the distance from the lowest point of the action portion 61 of the plunger 60 to the upper end of the inclined portion 61f and L2 is the distance from the lowest point of the lens body 2a to the front surface end of the lens body 2a. When, for example, the plunger 60 and the intraocular lens 2 are mounted on the rails 10f, 10f shown in FIG. 7, distances from a reference surface (for example, the horizontal plane including the upper end of the rails 10f, 10f may be used as the reference surface) in such a state may be set as P2, L2 and compared.

Also in the present embodiment, as shown in FIG. 8, a height P1 from a predetermined reference surface to the top surface of the protrusion 61e is set to be lower than a height L1 of the back surface end of the intraocular lens 2 in a state of being mounted on the stage portion 12 after the positioning member 50 being removed from the reference surface. In general, the support 2b is formed within the same height range as the lens end face 2d in a form in which the lens end face 2d is extended in the horizontal direction and accordingly, when the plunger 60 is pushed, an undersurface of the lens body 2a can be mounted on the protrusion 61e more reliably.

### <Second embodiment>

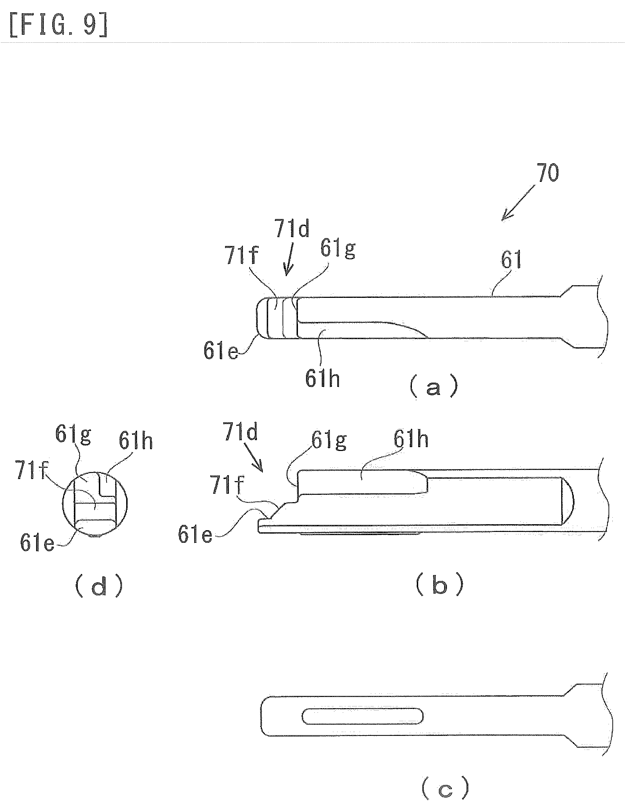

Next, the second embodiment of the present invention will be described. In the second embodiment, an example in which the inclined portion of the plunger is inclined only in the forward and backward direction of the plunger and is not inclined in the left and right direction of the plunger will be described. FIG. 9 shows an outline configuration of a tip 71d of a plunger 70 in the present embodiment. FIG. 9(a) is a plan view, FIG. 9(b) is the left side view, FIG. 9(c) is a bottom view, and FIG. 9(d) is a front view viewed from the front.

In FIG. 9, the same reference signs of the plunger 60 are attached to the same components as those in a tip portion of the plunger 60 in the first embodiment shown in FIG. 6 and the description thereof is omitted. As is evident from FIGS. 9 (a), 9(b), and 9 (d), an inclined portion 71f in the present embodiment is inclined only in the forward and backward direction of the plunger 70 and is not inclined in the left and right direction of the plunger 70. Even in such a shape, when, as shown in FIG. 7(b), the support 2b on the back side of the lens body 2a is brought into contact with the inclined portion 71f, the contact portion rises upward with a forward movement by pushing of the plunger 70. Then, the contact portion of the support 2b and the inclined portion 71f becomes higher than the linking portion 2c of the lens body 2a and the support 2b and thus, the support 2b is deformed so that the tip of the support 2b becomes still higher.

As a result, in the present embodiment, like in the first embodiment, by a simple operation of pushing the plunger 70, the support 2b is deformed such that the tip of the support 2b on the back side of the lens body 2a is folded up on the upper surface of the lens body 2a and then, the lens body 2a is deformed so as to be convex downward to be able to release the lens body 2a and the support 2b into the eyeball from the tip portion 10a of the nozzle portion 15. As a result, inconveniences such as wasting time to adjust the posture of the support after the insertion without being able to control the posture of particularly the support on the back side during insertion of the intraocular lens into an eyeball can be avoided.

### <Third embodiment>

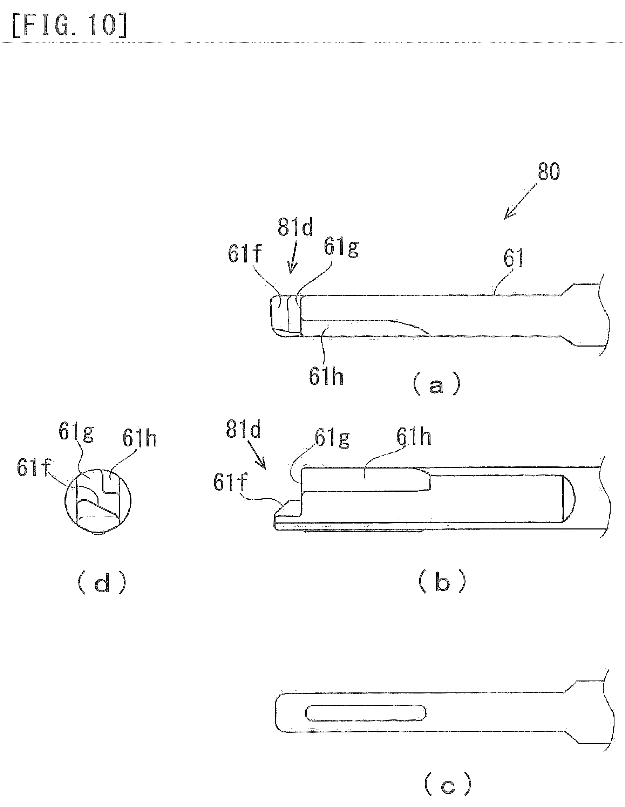

Next, the third embodiment of the present invention will be described. In the third embodiment, an example in which the inclined portion at the tip of the plunger is inclined in the forward and backward direction and the left and right direction of the plunger and no protrusion in a flat plate shape will be described. FIG. 10 shows an outline configuration of a tip 81d of a plunger 80 in the present embodiment. FIG. 10(a) isaplanview, FIG. 10 (b) is the left side view, FIG. 10 (c) is a bottom view, and FIG. 10(d) is a front view viewed from the front.

In FIG. 10, the same reference signs of the plunger 60 are attached to the same components as those in a tip portion of the plunger 60 in the first embodiment shown in FIG. 6 and the description thereof is omitted. As is evident from FIG. 10, the tip 81d of the plunger 80 in the present embodiment is not provided with a protrusion in a flat plate shape that gets into the underside of the lens body 2b. That is, in the present embodiment, when the plunger 80 is pushed, no protrusion will get into a gap between the lens body 2a and the set surface 12a.

In the initial state as shown, for example, in FIG. 7(a), however, consideration is given such that the support 2b on the back side of the lens body 2a is supported by the tip 81d of the plunger 80 and in such a case, even if there is no protrusion, by pushing the plunger 80, the support 2b on the back side of the lens body 2a can be brought into contact with an inclined portion 81f and deformed upward. Then, the support 2b is deformed upward by the tip 81d of the plunger 80 and deformed forward by the wall portion 61g so that the support 2b can be folded up on the upper surface of the lens body 2a.

According to the present embodiment, while the shape of the tip 81d of the plunger 80 is made still simpler, like in the first embodiment, by a simple operation of pushing the plunger 80, the support 2b on the back side of the lens body 2a is deformed so as to be folded up on the upper surface of the lens body 2a and then, the lens body 2a is deformed so as to be convex downward to be able to release the lens body 2a and the support 2b into the eyeball from the tip portion 10a of the nozzle portion 15.

In the first to third embodiments described above, an example in which the plunger includes the support relief groove 61h is described, but the support relief groove 61h is not necessarily required to obtain a minimum effect according to the present invention. In the first to third embodiments, without the support relief groove 61h, while the support 2b on the back side of the lens body 2a is deformed so as to be folded up on the upper surface of the lens body 2a, the lens body 2a can be deformed so as to be convex downward and the lens body 2a and the support 2b can be released into the eyeball from the tip portion 10a of the nozzle portion 15.

The above embodiments are described by taking a one-piece type intraocular lens in which the lens body 2a and the supports 2b, 2b of the intraocular lens 2 are integrally formed as an example, but it is needless to say that the present invention can be applied to a three-piece type intraocular lens in which the supports 2b, 2b are formed from a different member from that of the lens body 2a. In addition to the preset type distributed by setting the intraocular lens 2 to the stage portion 12, the present invention can also be applied to an intraocular lens-inserting instrument of the type used by the intraocular lens 2 being set by the operator to the stage portion 12 before an operation.

### Reference Signs List

- 1: Inserting instrument
- 2: Intraocular lens
- 10: Nozzle body
- 10a: Tip portion
- 10b: Back end portion
- 12: Stage portion
- 13: Stage cover portion
- 15: Nozzle portion
- 30, 60, 70, 80: Plunger
- 31, 61: Action portion
- 50: Positioning member
- 61d, 71d, 81d: Tip
- 61e: Protrusion
- 61f, 71f: Inclined portion
- 61g: Wall portion
- 61h: Support relief groove

## Claims

1. An intraocular lens-inserting instrument that inserts an intraocular lens into an eyeball by releasing the intraocular lens into the eyeball from a tip of an insertion cylindrical portion inserted into the eyeball through an incision, the inserting instrument comprising:
an instrument main body having the insertion cylindrical portion inserted through the incision formed in an eyeball tissue and formed into a substantially tubular shape;
an housing portion provided integrally with or separately from the instrument main body and capable of arranging the intraocular lens in the instrument main body by accommodating the intraocular lens including a lens body and a plurality of supports that support the lens body in the eyeball; and
a plunger that releases the intraocular lens from a tip of the insertion cylindrical portion into the eyeball by pressing the intraocular lens accommodated in the housing portion by the tip by being pushed into the instrument main body, wherein
the intraocular lens is arranged in the instrument main body such that one support is arranged on a back side in a pressing direction of the lens body by the plunger,
an inclined plane inclined toward an optical axis front side of the lens body from a front side to the back side in the pressing direction is provided at the tip of the plunger, and
when the intraocular lens is pressed by the tip of the plunger, the tip of the support arranged on the back side in the pressing direction of the lens body is caused to be arranged on the optical axis front side of a surface on the optical axis front side of the lens body by deforming the support to the optical axis front side of the lens body by the inclined plane and also the support is deformed by the inclined plane to the front side in the pressing direction to be able to be folded up such that the support overlaps with the surface on the optical axis front side of the lens body when viewed from an optical axis side.

2. The intraocular lens-inserting instrument according to claim 1, wherein the inclined plane is inclined toward the optical axis front side of the lens body from a root side to a tip side of the support arranged on the back side in the direction perpendicular to the pressing direction and the optical axis of the lens body.

3. The intraocular lens-inserting instrument according to claim 1 or 2, wherein a wall portion formed perpendicularly with respect to the pressing direction to move the lens body to the front side in the pressing direction and also to deform the support arranged on the back side in the pressing direction of the lens body to the front side in the pressing direction is provided on the back side in the pressing direction of the inclined plane at the tip of the plunger.

4. The intraocular lens-inserting instrument according to any one of claims 1 to 3, wherein a recess that accommodates a linking portion of the support that is folded up and the lens body in a state in which the support arranged on the back side in the pressing direction of the lens body is folded up on the surface on the optical axis front side of the lens body so as to overlap therewith when viewed from the optical axis side is provided at the tip of the plunger.

5. The intraocular lens-inserting instrument according to anyone of claims 1 to 4, wherein a projecting portion projecting to the front side in the pressing direction from the inclined plane to make a portion of the lens body mountable thereon is provided on an optical axis back side of the lens body of the inclined plane at the tip of the plunger.

6. The intraocular lens-inserting instrument according to any one of claims 1 to 5, wherein the inclined plane is provided such that an end on the optical axis front side of the inclined plane is arranged on the optical axis front side from an end face of the surface on the optical axis front side of the lens body.
